# EUROPEAN PATENT APPLICATION

(11) **EP 1 933 187 A1**
(43) Date of publication of application: **18.06.2008**
(21) Application number: 07122593.2
(22) Date of filing: 07.12.2007
(51) Int. Cl.: G02B 21/00, G02B 21/06, A61B 3/00, A61B 3/13

(54) **Illumination system for surgical microscope**

(30) Priority: 07.12.2006 US 868959 P
(71) Applicant: Alcon RefractiveHorizons, Inc., Fort Worth, Texas 76134 (US)
(72) Inventor: HOPLER, Mark D., Fort Worth, TX 76108 (US); CAMPIN, John A., Orlando, FL 32828 (US); SOSKIND, Yakov, Plainsboro, NJ 08536 (US)
(74) Representative: Hanna, Peter William Derek

(57) **Abstract**

An illumination system (301) and method of illuminating a surgical site are disclosed. One embodiment of the illumination system comprises: a composite illumination source (302), comprising a plurality of light sources operable to emit a plurality of collinear light beams (310); and an illumination lens (304), operable to receive and focus the plurality of light beams at a focal plane; wherein the plurality of collinear light beams are directed onto an off-axis portion (306) of the illumination lens and wherein the illumination lens is aligned co-axially and confocally with an objective lens (305) of an observation device. The light sources can be solid-state lighting devices, such as light-emitting diodes. The observation device can be a surgical ophthalmic microscope. The illumination lens can have a larger diameter than the objective lens, and the off-axis portion of the illumination lens can comprise a Fresnel type lens. The illumination lens can have a clear central portion (307) of a diameter sufficient to prevent interference with the focusing function of the objective lens, or the illumination lens can comprise a ring with a central opening of a diameter sufficient to prevent interference with the focusing function of the objective lens (305).

## Description

### FIELD OF INVENTION

The present invention relates to the field of surgical illumination. More specifically, it relates to illumination systems used in ophthalmic surgical microscopes to illuminate a surgical observation area. Even more specifically, the present invention relates to ophthalmic illumination systems that reduce the risk of injury to a patient's eye from a light source.

### BACKGROUND

An illumination system is an integral and important part of a surgical microscope used to make visible the details of an object or area during surgery. Ophthalmic instruments, including ophthalmic surgical microscopes, not only require sufficient levels of irradiance at an object (e.g., the eye plane) for visualization purposes, but are also required to comply with eye safety requirements. These safety requirements impose a critical constraint, assuring that no damage is done to a patient's eye due to illumination during surgery.

Recent developments in solid-state lighting technology make light-emitting diode (LED) sources an attractive alternative to traditional incandescent or halogen light sources. LEDs are more efficient, have longer operating life and lower heat dissipation than these traditional sources. However, the emission spectra of these LED sources differ from that of incandescent or halogen light sources and may contain a higher fraction of shorter wavelength radiation that is more harmful to the eye than light from the longer wavelength part of the spectrum, as defined in ANSI/IESNA RP-27.1-96 "Recommended Practice for Photobiological Safety for Lamps & Lamp Systems - General Requirements" and in the ISO 10936-2 standard "Optics and optical instruments - Operating microscopes - Part 2: Light hazard from operating microscopes used in ocular surgery."

Reduction in the phototoxicity of the illumination light provided by LED (or other light sources) may be achieved by filtering out the hazardous spectral composition of the light source in a manner as disclosed in US Patent Application 2005/0117209 "Surgical Microscope for Ophthalmology" published on June 2, 2005, and assigned to Carl Zeiss AG. However, while this and similar techniques can be readily applied to reduce the content of the invisible ultraviolet (UV) radiation from a light source, whenever applicable, filtering out a spectral portion of the light source's visible radiation will inevitably result in undesirable changes in color temperature and perception of the illumination light. Reduction in the blue-light content of the illumination beam will cause "yellowing" of the illumination pattern and an associated reduction in the perceived observation contrast at the site under observation. It is therefore desirable to have as high a color temperature of the illumination light source as possible, while providing a sufficient safety margin with respect to retinal exposure levels.

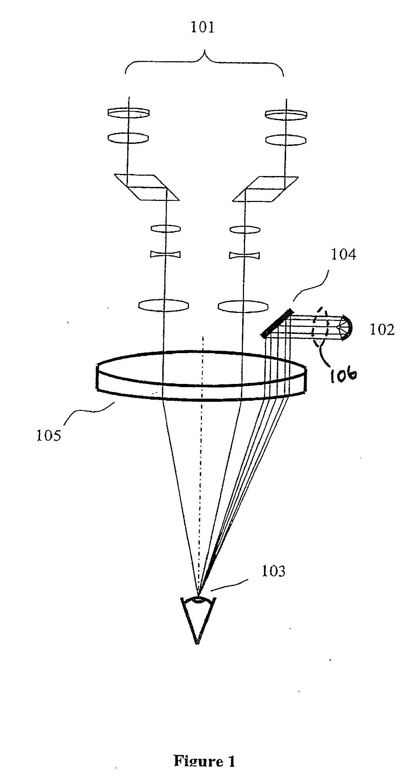

Eye hazard reduction for a given irradiance in the eye plane may also be achieved by enlarging the effective size of the illumination source through the use of oblique illumination, effectively reducing the flux density at the surface of, for example, the retina. A common way of introducing an oblique illumination into a surgical microscope system is by off-axis propagation of the light through the microscope objective lens, as is shown schematically in FIG. 1. This technique is well documented in the art. As is shown in FIG. 1, the illumination beam 106 is converging to the focal plane 103 of the microscope objective lens 105, where the image is being observed.

Due to the limited lateral dimensions of the microscope objective lens 105 in a typical ophthalmic surgical microscope, only a limited number of light sources 102 can be used to create an oblique illumination beam in this way. This limitation limits the angular extent of the light source 102 as viewed from the eye plane, limiting also the minimum achievable flux density at the surface of the retina.

An alternative way of achieving an oblique illumination of a surgical site is through the use of direct illumination in which multiple beams 206 are directed at an angle with respect to the eye plane, geometrically converging at the observation plane 203, as shown schematically in FIG. 2 and disclosed in US Patent Application 2004/0174592 "Surgical Microscope Having An Object Field Illumination System" published on September 9, 2004, and assigned to Leica Microsystems AG, and US Patent 5,155,509 "Oblique Illumination Device for Use With An Ophthalmic Microscope," issued October 13, 1992, and assigned to Storz Instrument Co.

Direct illumination of this kind has several drawbacks, including the complexity of the opto-mechanical assembly and the need for individual beam alignment and aiming in three-dimensional space. Direct illumination also precludes achieving a continuous extended illumination source of significant lateral dimensions by concatenating individual smaller-size sources 202.

It would therefore be desirable to provide an illumination system for a surgical microscope comprising at least one extended light source that provides a desired irradiance at an eye plane and has significant angular dimensions that are necessary to decrease the light density on the retina, thus reducing the retinal exposure level and the accompanying hazard level.

It would be desirable to provide an illumination system for a surgical microscope that is easy to fabricate and does not require the alignment complexities associated with prior art direct oblique illumination.

It would also be further desirable to provide an illumination system with elevated color temperature for enhanced contrast imagery through a surgical microscope.

### Summary of the Invention

The present invention provides an illumination system in accordance with claims which follow. In view of the foregoing, it is an object of the embodiments of this invention to provide a method for extending the angular dimensions of an illumination system in an ophthalmic surgical microscope to effectively reduce the light density on the retina for a given level of irradiance in the eye plane.

It is another object of the embodiments of this invention to provide an illumination system for a surgical microscope that comprises at least one extended light source for oblique illumination at the eye plane with reduced retinal exposure.

It is still another object of the embodiments of the present invention to provide an illumination system for a surgical microscope that is simpler to fabricate than prior art illumination systems.

The above objects can be achieved, for example, by arranging or clustering several individual smaller size illumination sources on a substrate, such as a printed circuit board (PCB), to extend the effective size of the illumination source as viewed from the eye plane. Beam-forming optics, reflective and/or refractive, can be employed to form individual collinear beams associated with each of the light sources. Some of the light sources may employ the beam-forming optics as an integral part. In a preferred embodiment, individual beams can be concatenated so that no dark space is left between the beams.

In one embodiment, an auxiliary larger size illumination lens can be employed to achieve oblique illumination. The output of each individual light source is directed onto the auxiliary lens parallel to the lens optical axis with varying lateral offsets defined by the spatial location of the individual source with respect to the system optical axis. The extent of the lateral offset defines the obliquity of the angle of the beam at the eye plane. Such an illumination system can comprise: a composite illumination source, comprising a plurality of light sources operable to emit a plurality of collinear light beams; and an auxiliary illumination lens, operable to receive and focus the plurality of light beams at a focal plane; wherein the plurality of collinear light beams are directed onto an off-axis portion of the illumination lens and wherein the illumination lens is aligned co-axially and confocally with an objective lens of an observation device. The light sources can be solid-state lighting devices, such as light-emitting diodes. The observation device can be an ophthalmic surgical microscope. The illumination lens can have a larger diameter than the objective lens, and the off-axis portion of the illumination lens can comprise a Fresnel type lens. The illumination lens can have a clear central portion of a diameter sufficient to prevent interference with the focusing function of the objective lens, or the illumination lens can comprise a ring with a central opening of a diameter sufficient to prevent interference with the focusing function of the objective lens (i.e., a central portion of the illumination lens can be removed).

The features of the present invention, including construction and operational details, will now be more particularly described with reference to the accompanying drawings.

### Brief Description of the Drawing

A more complete understanding of the present invention and the advantages thereof may be acquired by referring to the following description, taken in conjunction with the accompanying drawings in which like reference numbers indicate like features and wherein:
FIG. 1 is a schematic representation of an ophthalmic surgical microscope with oblique illumination achieved by off-axis propagation through the microscope objective lens;

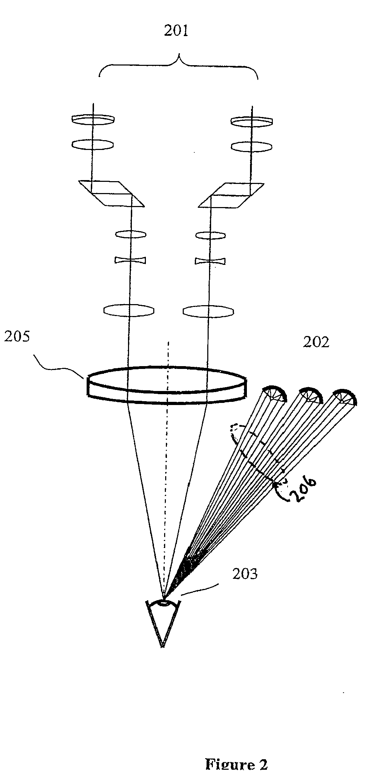

FIG. 2 is a schematic representation of an ophthalmic surgical microscope with oblique illumination achieved through the use of direct illumination; and
FIG. 3 is a schematic representation of an ophthalmic surgical microscope with extended illumination source and oblique illumination achieved through the use of an auxiliary lens in accordance with an embodiment of the present invention.

### Detailed Description of the Invention

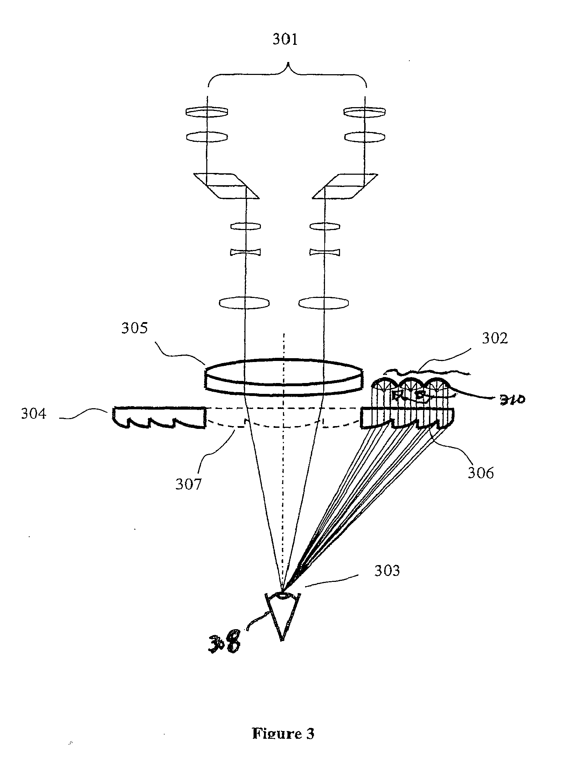

FIG. 3 is a schematic representation of an ophthalmic surgical microscope 301 with extended illumination source 302 forming an oblique illumination pattern at an eye plane 303 achieved through the aid of an auxiliary lens 304 in accordance with an embodiment of the present invention. Auxiliary lens 304 is a larger lens than the main objective lens 305.

The extended size illumination source with oblique illumination pattern at the eye plane 303 and reduced light density on the retina is obtained in this embodiment by arranging several individual smaller size light sources 310 in a specific pattern or cluster on a substrate such as a printed circuit board (PCB) to form a composite extended illumination source 302. The individual light sources 310 are preferably solid-state lighting devices such as light-emitting diodes (LEDs). Spectral composition of the LEDs is chosen based on desired illumination requirements. For surgical microscopes, including ophthalmic surgical microscopes, so called white light LEDs emitting in a broad range of the visible spectrum can be used.

Some types of LEDs have optics, such as a lens, as an integral part of the LED device and emit a well-defined output optical beam. Some other types of LEDs emit in a broad range of angles and require beam-forming optics to confine or collimate the output beam. In the latter case, beam-forming optics (not shown), as will be known to those having average skill in the art, may be conveniently arranged in a pattern or cluster reflecting the arranged pattern of the individual LEDs 310.

The light beams from the individual light sources 310 are emitted collinear to each other and are directed onto a larger size auxiliary lens 304 with an offset with respect to the lens 304 optical axis. The auxiliary lens 304 is aligned co-axially with respect to the main objective lens 305 of the microscope 301 such that the optical axes of the two lenses coincide. The auxiliary lens 304 is also aligned with respect to the main objective lens 305 of the microscope 301 in a confocal manner such that the focal planes of the two lenses coincide. In a preferred embodiment, auxiliary lens 304 can be a Fresnel type lens, as shown schematically in FIG. 3. Off-axis portion 306 of the lens 304 is primarily used for illumination. The central portion 307 of the Fresnel lens 304 can be optically clear or can be removed, allowing the microscope main objective lens 305 to perform its functions in that area.

Individual beams from the composite extended illumination source 302 propagate through the off-axis portion 306 of the Fresnel lens 304 and are directed towards a focal point of the lens 304 coinciding with the eye plane 303. The beams form a continuum of oblique angles that define an illumination pattern at the eye plane 303. A fraction of the light in the eye plane 303 propagates through the pupil of the eye 308 and forms an illumination pattern on the retina. The large angular dimensions of the extended illumination source 302 spread the fraction of illumination light over an extended area of the retina, effectively reducing the retinal exposure levels as well as the risk of injury to the eye 308 from the illumination source 302.

Reduced retinal exposure levels allow the increase of the correlated color temperature of the illumination system 301 by increasing the relative content of the shorter wavelength radiation in the spectral distribution of the light sources 310 while complying with eye safety requirements. The increase in the correlated color temperature of the extended illumination source 302 leads to improved image contrast at the observed surgical site as viewed through the microscope.

Although the present invention has been described in detail herein with reference to the illustrated embodiments, it should be understood that the description is by way of example only and is not to be construed in a limiting sense. It is to be further understood, therefore, that numerous changes in the details of the embodiments of this invention and additional embodiments of this invention will be apparent to, and may be made by, persons of ordinary skill in the art having reference to this description. It is contemplated that all such changes and additional embodiments are within the scope of this invention as claimed below.

## Claims

1. An illumination system (301), comprising:
a composite illumination source (302), comprising a plurality of light sources operable to emit a plurality of collinear light beams(310); and
an illumination lens (304), operable to receive and focus the plurality of light beams at a focal plane;
wherein the plurality of collinear light beams are directed onto an off-axis portion (306) of the illumination lens and wherein the illumination lens is aligned co-axially and confocally with an objective lens (305) of an observation device.

2. The illumination system of Claim 1, wherein the light sources (302) are solid-state lighting devices.

3. The illumination system of Claim 2, wherein the solid-state lighting devices are light-emitting diodes.

4. The illumination system of Claim 1, wherein the observation device is an ophthalmic surgical microscope.

5. The illumination system of Claim 1, wherein the illumination lens (304) has a larger diameter than the objective lens, and wherein the off-axis portion (306) of the illumination lens comprises a Fresnel lens and a central portion (307) of the illumination lens sufficient to prevent interference with the focusing function of the objective lens (305) is removed.
